# EUROPÄISCHE PATENTSCHRIFT

(11) **EP 1 093 762 B1**
(45) Veröffentlichungstag und Bekanntmachung des Hinweises auf die Patenterteilung: **29.08.2007**
(21) Anmeldenummer: 00121355.2
(22) Anmeldetag: 11.10.2000
(51) Int. Cl.: A61B 17/70, A61B 17/80

(54) **Knochenplatte**
Bone plate
Plaque de fixation d'os

(30) Priorität: 18.10.1999 DE 19950270
(43) Veröffentlichungstag der Anmeldung: 25.04.2001
(73) Patentinhaber: DePuy Spine Sàrl, 2400 Le Locle (CH)
(72) Erfinder: Schäfer, Bernd, 6315 Oberägeri (CH); Halm, Henry, Dr., 49143 Bissendorf (DE)
(74) Vertreter: Dreiss, Fuhlendorf, Steimle & Becker

(56) Entgegenhaltungen:
- WO-A-97/09000
- DE-A- 4 414 782
- DE-A- 4 433 360
- DE-A- 19 509 331
- US-A- 5 275 601

## Beschreibung

Die Erfindung betrifft eine Osteosyntheseeinrichtung mit einer Knochenplatte.

Knochenplatten sind allgemein, z.B. aus der DE-A-44 33 360 bekannt. Derartige Knochenplatten werden z.B. an Wirbeln befestigt, um die Wirbel zu stabilisieren. Hierfür werden die einzelnen Knochenplatten über Stäbe miteinander verbunden, wobei die Stäbe an den Knochenplatten befestigt, insbesondere festgeklemmt werden. Zum Befestigen der Knochenplatten an den Wirbeln werden Knochenschrauben verwendet, die die Knochenplatten durchdringen und in den Wirbel eingeschraubt werden. Die Knochenplatte wird in der Regel mittels des Schraubenkopfes festgehalten.

Dabei hat sich gezeigt, dass anfänglich die Knochenplatte zwar fest am Wirbel anliegt, mit der Zeit jedoch Lockerungen auftreten können, insbesondere wenn sich die Knochenschraube im Wirbel lockert oder wenn sich der Knochen in dem Bereich, in welchem die Knochenplatte anliegt, verändert.

Aus der US-A-5,275,601 ist eine Knochenschraube und eine Knochenplatte mit einer Aufnahmeöffnung für den Kopf der Knochenschraube bekannt. Sowohl die Aufnahmeöffnung für den Kopf der Knochenschraube als auch der Kopf der Knochenschraube sind mit in Axialrichtung verlaufenden Rillen versehen, wobei die Rillen eine Sägezahnstruktur aufweisen. Die Sägezahnstruktur ist so geformt, dass das Ausschrauben blockiert wird.

Eine derartige Anordnung besitzt zwar den Vorteil, dass die Schraube gegen unbeabsichtigtes Lockern oder Lösen gesichert ist. Jedoch wird aufgrund der sowohl am Kopf der Schraube als auch an der Innenfläche der Aufnahmeöffnung für den Kopf der Knochenschraube vorgesehenen Rillen das Einschrauben der Schraube stark erschwert, da entweder das Material der Schraube oder das Material der Platte verformt werden muss.

Der Erfindung liegt daher die Aufgabe zugrunde, eine Osteosyntheseeinrichtung bereitzustellen, bei welcher die Gefahr einer späteren Lockerung verringert ist.

Diese Aufgabe wird erfindungsgemäß mit einer Osteosyntheseeinrichtung gelöst, die die Merkmale des Anspruchs 1 aufweist.

Aufgrund der Oberflächenstruktur in der Aufnahmeöffnung, an welcher der Schraubenkopf anliegt, wird eine Rückhaltewirkung, d.h. Ausdrehrichtung für den Schraubenkopf erzielt. Die Knochenplatte wird dadurch nicht nur kraftschlüssig sondern auch formschlüssig mit dem Schraubenkopf und somit mit der Schraube verbunden. Aufgrund des Formschlusses wird die Gefahr verringert, dass sich die Schraube lockert, d.h. den festen Halt im Knochen verliert. Außerdem besteht nach wie vor ein Verbund zwischen Schraubenkopf und Knochenplatte, auch wenn der Knochen im Bereich der Anlage an der Knochenplatte seine Form verändert.

Bevorzugt ist die Aufnahmeöffnung kreisrund ausgebildet. Derartige Öffnungen erlauben ein problemloses Einschrauben und Anliegen des Schraubenkopfes.

Die Erfindung sieht vor, dass der vom Knochen abgewandte Bereich der Aufnahmeöffnung eine Oberflächenstruktur aufweist. Insbesondere bei kalottenförmig ausgebildeten Aufnahmeöffnungen, in welche ein ballig ausgebildeter Kopf der Knochenschraube eingesetzt wird, ist der nahezu senkrecht verlaufende, d.h. weniger geneigte, vom Knochen abgewandte Bereich der Aufnahmeöffnung mit der Oberflächenstruktur versehen, wodurch der Halt des Schraubenkopfes sicherer ist als im geneigten Bereich. In dem Bereich, welcher im wesentlichen senkrecht zur Achse der Schraube ausgerichtet ist, bewegt sich der Schraubenkopf beim Einschrauben der Schraube im wesentlichen parallel zur und entlang der Innenoberfläche der Aufnahmeöffnung. Erst unmittelbar am Ende des Einschraubvorganges legt sich der Schraubenkopf mit seinem unteren Bereich auf den geneigten Abschnitt der kalottenförmig ausgeführten Aufnahmeöffnung auf und hält dadurch die Knochenplatte am Knochen fest.

Bevorzugt weist die Oberflächenstruktur einen in Umfangsrichtung gerichteten Strukturverlauf auf. Auf diese Weise wird eine Hemmung in Umfangsrichtung, d.h. in Drehrichtung der Schraube erzielt.

Bevorzugte Ausführungsformen sehen vor, dass die Oberflächenstruktur in Form von Längsnuten, einer Verzahnung, einer Riffelung oder dgl. ausgebildet ist. Es ist auch denkbar, dass die Oberflächenstruktur durch eine Aufrauung der Oberfläche geschaffen wird.

Eine bevorzugte Ausführungsform wird darin gesehen, dass die Längsnuten oder die Verzahnung in Form einer Sägeverzahnung ausgebildet ist. Dabei weist jeder Sägezahn der Sägeverzahnung eine steile und eine flache Flanke auf. Um eine Hemmwirkung in Aufschraubrichtung der Schraube zu erzielen, steigt die flache Flanke in Einschraubrichtung der Knochenschraube an. Die Knochenschraube kann daher relativ leicht eingeschraubt werden und wird von der steilen Flanke des Sägezahns der Sägeverzahnung gegen ein Ausschrauben gesichert.

Eine Optimierung der Hemmwirkung wird dadurch erzielt, dass der Schraubenkopf mit einer Oberflächenstrukturierung versehen ist, die die Hemmwirkung unterstützt. Insbesondere kann der Schraubenkopf ebenfalls mit in Längsrichtung verlaufenden Nuten oder dgl. versehen sein. Auch eine Sägeverzahnung ist denkbar.

Weitere Vorteile, Merkmale und Einzelheiten der Erfindung ergeben sich aus der nachfolgenden Beschreibung, in der unter Bezugnahme auf die Zeichnung ein besonders bevorzugtes Ausführungsbeispiel im einzelnen beschrieben ist.

In der Zeichnung zeigen:
- Figur 1: eine perspektivische Darstellung einer Knochenplatte gemäß der Erfindung; und
- Figur 2: einen Ausschnitt der Knochenplatte, die mit einer Oberflächenstruktur versehene Aufnahmeöffnung in Draufsicht zeigt.

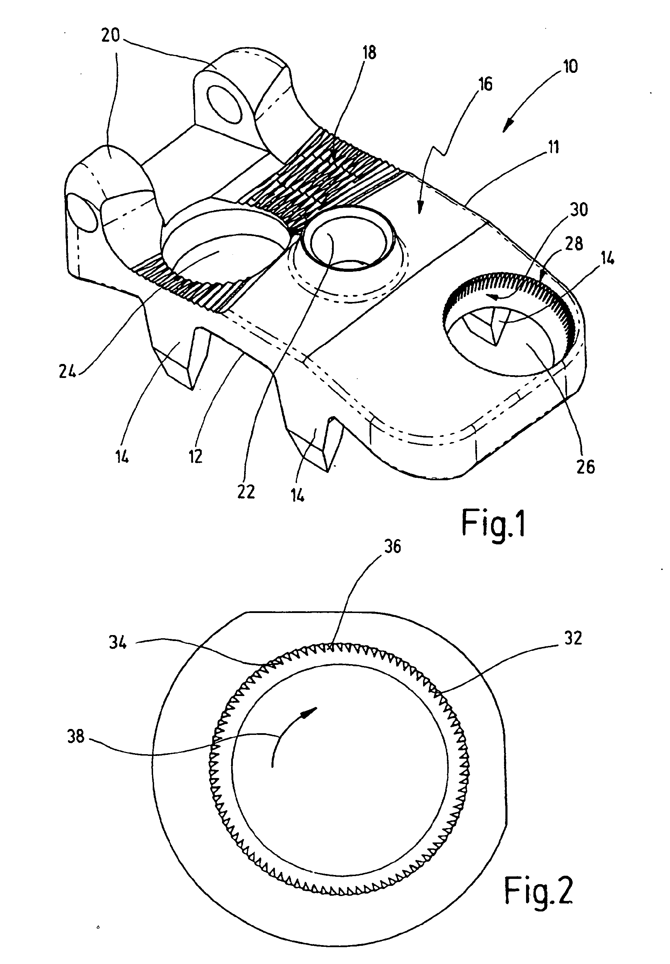

In der Figur 1 ist von einer Knochenplatte 10 ein Grundkörper 11 dargestellt, der an seiner z.B. einem Wirbel zugewandten Unterseite 12 mit Ankerkeilen 14 versehen ist, welche in den Knochen eingeschlagen werden. Der Grundkörper 11 liegt dann mit ihrer Unterseite 12 auf der Oberfläche des Knochens auf. An ihrer Oberseite 16 weist der Grundkörper 11 einen Aufnahmebereich 18 auf, in welchen ein Fixierstab (nicht dargestellt) eingelegt wird. Dieser Fixierstab wird mittels eines Deckels, der an einem Scharnier 20 schwenkbar am Grundkörper 11 festgelegt ist, fixiert, indem der Deckel auf den Fixierstab aufgeschwenkt und z.B. mittels einer Schraube, welche in eine Gewindebohrung 22 eingeschraubt wird, gehalten wird. Der Grundkörper 11 weist außerdem zwei Aufnahmeöffnungen 24 und 26 auf, in welche Knochenschrauben eingesetzt werden, die ihrerseits in den Knochen eingeschraubt werden. Auf diese Weise wird der Grundkörper 11 am Knochen fixiert.

Die Aufnahmeöffnung 26 ist kalottenförmig ausgeführt, so dass sie einen ballig ausgebildeten Schraubenkopf aufnehmen kann. Der der Oberseite 16 zugewandte Bereich der Aufnahmeöffnung 26 ist dabei im wesentlichen steil und der der Unterseite 12 zugewandte Bereich der Aufnahmeöffnung 26 geringfügig geneigt ausgebildet.

Die Aufnahmeöffnung 26 weist in ihrem oberen, d.h. der Oberseite 16 zugewandten Bereich eine Oberflächenstruktur 28 auf. Aufgrund dieser Oberflächenstruktur 28 ist die Aufnahmeöffnung 26 an ihrer Innenoberfläche 30 nicht mehr glatt sondern strukturiert. Diese Oberflächenstruktur 28 ist in Form einer Sägeverzahnung 32 ausgebildet, die sich aus Figur 2 ergibt. Diese Sägeverzahnung 32 besitzt eine flache Flanke 34 und eine steile Flanke 36. Dabei ist die flache Flanke 34 derart angeordnet, dass sie in Einschraubrichtung (Pfeil 38) radial ansteigt. Dies erleichtert ein Einschrauben der Knochenschraube, da die Hemmwirkung in dieser Einschraubrichtung 38 wesentlich geringer ist als in Ausschraubrichtung, in welcher die steile Flanke 36 radial ansteigt. Die steile Flanke 36 übt also ihre Hemmwirkung in Ausschraubrichtung aus und verhindert somit ein selbsttätiges Ausschrauben der Knochenschraube bzw. eine Lockerung der Knochenschraube im Knochen. Der Schraubenkopf der Knochenschraube ist nach dem Einschrauben formschlüssig in der Aufnahmeöffnung 26 verankert.

Selbstverständlich kann auch die Aufnahmeöffnung 24 mit einer derartigen Oberflächenstruktur 28 versehen sein. Auch kann der Schraubenkopf partiell oder über seinen gesamten Außenumfang mit einer Oberflächenstruktur, insbesondere einer Sägeverzahnung versehen sein, die passend zur Sägeverzahnung 32 der Oberflächenstruktur 28 der Aufnahmeöffnung 26 ausgerichtet ist.

## Patentansprüche

1. Osteosyntheseeinrichtung mit einer Knochenplatte (10) für die Osteosynthese mit einem plattenförmigen Grundkörper (11) und einem Deckel, wobei der Grundkörper (11) wenigstens eine Aufnahmeöffnung (24, 26) zur Aufnahme einer diesen z.B. an einem Wirbel befestigenden Knochenschraube aufweist, indem der Schraubenkopf der Knochenschraube von der Aufnahmeöffnung (24, 26) aufgenommen wird, wobei die Aufnahmeöffnung (26) einen dem Knochen zugewandten und einen vom Knochen abgewandten Bereich aufweist, **dadurch gekennzeichnet, dass** der vom Knochen abgewandte Bereich der Aufnahmeöffnung (26) an ihrer dem Schraubenkopf zugewandten Fläche (30) mit einer Oberflächenstruktur (28) versehen ist und der dem Knochen zugewandte Bereich der Aufnahmeöffnung (26) glatt ausgebildet ist.

2. Osteosyntheseeinrichtung nach Anspruch 1, **dadurch gekennzeichnet, dass** die Aufnahmeöffnung (24, 26) kreisrund ist.

3. Osteosyntheseeinrichtung nach einem der vorhergehenden Ansprüche, **dadurch gekennzeichnet, dass** die Oberflächenstruktur (28) sich über den gesamten Innenumfang der Aufnahmeöffnung (26) erstreckt.

4. Osteosyntheseeinrichtung nach einem der vorhergehenden Ansprüche, **dadurch gekennzeichnet, dass** die Oberflächenstruktur (28) einen in Umfangsrichtung gerichteten Strukturverlauf aufweist.

5. Osteosyntheseeinrichtung nach einem der vorhergehenden Ansprüche, **dadurch gekennzeichnet, dass** die Oberflächenstruktur (28) in Form von Längsnuten, einer Verzahnung oder einer Riffelung ausgebildet ist.

6. Osteosyntheseeinrichtung nach Anspruch 5, **dadurch gekennzeichnet, dass** die Längsnuten oder Verzahnung in Form einer Sägeverzahnung (32) ausgebildet ist.

7. Osteosyntheseeinrichtung nach Anspruch 6, **dadurch gekennzeichnet, dass** jeder Sägezahn der Sägeverzahnung (32) eine steile Flanke (36) und eine flache Flanke (34) aufweist.

8. Osteosyntheseeinrichtung nach Anspruch 7, **dadurch gekennzeichnet, dass** die flache Flanke (34) in Einschraubrichtung (38) der Knochenschraube ansteigt.

9. Osteosyntheseeinrichtung nach einem der vorhergehenden Ansprüche mit wenigstens einer Knochenschraube zur Befestigung des Grundkörpers z.B. an einem Wirbel, **dadurch gekennzeichnet, dass** der Schraubenkopf mit einer Oberflächenstrukturierung versehen ist.

10. Osteosyntheseeinrichtung nach Anspruch 9, **dadurch gekennzeichnet, dass** die beiden Oberflächenstrukturierungen derart ausgebildet sind, dass sie eine Hemmwirkung in einer Drehrichtung der Knochenschraube, insbesondere in Ausschraubrichtung bewirken.

## Claims

1. Osteosynthesis device with a bone plate (10) for the osteosynthesis having a plate-shaped basic body (11) and a lid, said basic body (11) having at least one opening (24, 26) for accepting a bone screw to attach the bone plate to e.g. vertebrae, by receiving the screw head of the bone screw by said opening (24, 26), wherein said opening (26) having an area facing the bone and an area facing away from the bone, **characterized in that** the area of the opening (26) facing away from the bone is provided with a surface structure at the area (30) facing the screw head and the area of the opening (26) facing the bone is manufactured smooth.

2. Osteosynthesis of claim 1, **characterized in that** opening (24, 26) is circular.

3. Osteosynthesis of one of the preceding claims, **characterized in that** said surface structure (28) extends along an entire inner periphery of said opening (26).

4. Osteosynthesis of one of the preceding claims, **characterized in that** said surface structure (28) extends in a peripheral direction.

5. Osteosynthesis of one of the preceding claims, **characterized in that** said surface structure (28) comprises longitudinal grooves, serrations or a fluting.

6. Osteosynthesis of claim 5, **characterized in that** said longitudinal grooves or serrations are formed by saw-tooth serrations (32).

7. Osteosynthesis of claim 5, **characterized in that** each saw tooth of said saw-tooth serrations (32) comprises a steep flank (36) and a flat flank (34).

8. Osteosynthesis of claim 7, **characterized in that** said flat flank (34) rises in a screwing-in direction (38) of the bone screw.

9. Osteosynthesis of one of the preceding claims having at least a bone screw for fastening the basic body e.g. to a vertebrae, **characterized in that** said screw head is provided with a surface structure.

10. Osteosynthesis of claim 9, **characterized in that** said two surface structures are structured to have a blocking effect in a turning direction of the bone screw, preferably in the unscrewing direction.

## Revendications

1. Dispositif d'ostéosynthèse avec une plaque de fixation d'os (10) pour l'ostéosynthèse avec un corps de base (11) en forme de plaque et avec un couvercle, le corps de base (11) présentant au moins une ouverture de réception (24, 26) pour recevoir un vis à os fixant celui-ci par exemple à une vertèbre, en ce sens que la tête de vis de la vis à os est reçue par l'ouverture de réception (24, 26), l'ouverture de réception (26) présentant une zone orientée vers l'os et une zone opposée à l'os, **caractérisé en ce que** la zone de l'ouverture de réception (26) qui est opposée à l'os est pourvue d'une structure de surface (28) sur sa surface (30) orientée vers la tête de vis et la zone de l'ouverture de réception (26) qui est orientée vers l'os est configurée de manière lisse.

2. Dispositif d'ostéosynthèse selon la revendication 1, **caractérisé en ce que** l'ouverture de réception (24, 26) est circulaire.

3. Dispositif d'ostéosynthèse selon l'une quelconque des revendications précédentes, **caractérisé en ce que** la structure de surface (28) s'étend sur tout le pourtour intérieur de l'ouverture de réception (26).

4. Dispositif d'ostéosynthèse selon l'une quelconque des revendications précédentes, **caractérisé en ce que** la structure de surface (28) présente un profil de surface dirigé dans le sens périphérique.

5. Dispositif d'ostéosynthèse selon l'une quelconque des revendications précédentes, **caractérisé en ce que** la structure de surface (28) est configurée en forme de rainures longitudinales, d'une denture ou d'un striage.

6. Dispositif d'ostéosynthèse selon l'une quelconque des revendications précédentes, **caractérisé en ce** les rainures longitudinales ou la denture est configurée en forme de denture de scie (32).

7. Dispositif d'ostéosynthèse selon la revendication 6, **caractérisé en ce que** chaque dent de scie de la denture de scie (32) présente un flanc raide (36) et un flanc plat (34).

8. Dispositif d'ostéosynthèse selon la revendication 7, **caractérisé en ce que** le flanc plat (34) s'élève dans le sens de visage (38) de la vis à os.

9. Dispositif d'ostéosynthèse selon l'une quelconque des revendications précédentes avec au moins une vis à os pour fixer le corps de base par exemple à une vertèbre, **caractérisé en ce que** la tête de vis est pourvue d'une structuration de surface.

10. Dispositif d'ostéosynthèse selon la revendication 9, **caractérisé en ce que** les deux structurations de surface sont configurées de telle manière qu'elles produisent un effet de blocage dans un sens de rotation de la vis à os, en particulier dans le sens de dévissage.
